# EUROPEAN PATENT APPLICATION

(11) **EP 3 834 862 A1**
(43) Date of publication of application: **16.06.2021**
(21) Application number: 20212979.7
(22) Date of filing: 10.12.2020
(51) Int. Cl.: A61M 5/142

(54) **TUBE DIAMETER RECOGNITION**

(30) Priority: 11.12.2019 US 201916710645
(71) Applicant: Avoset Health Ltd., 4250529 Netanya (IL)
(72) Inventor: EITAN, Boaz, Hofit 4029500 (IL)
(74) Representative: Evens, Paul Jonathan

(57) **Abstract**

Method and apparatus for (A) isolating a segment (22) of a fluid-filled conduit (24) by occluding a first site of the fluid-filled conduit and a second site of the fluid-filled conduit (B) incrementally squeezing a portion of the isolated segment of the fluid-filled conduit, (C) for each iteration of squeezing, measuring (i) an increase in force exerted by the isolated segment of the fluid-filled conduit or (ii) an increase in size of the isolated segment, associated with a respective pressure change during that incremental squeezing, and (D) measuring an indication of the size of the conduit when (i) an increase in force exerted by the isolated segment, measured in response to an incremental squeezing, passes above a threshold value, or (ii) an increase in size of the isolated segment, measured in response to an incremental squeezing, passes below a threshold value. Other applications are also described.

## Description

### CROSS-REFERENCE TO RELATED APPLICATIONS

The present application claims the priority of US 16/710,645 to Eitan, filed Dec. 11, 2019, entitled "Tube diameter recognition," and which is incorporated herein by reference.

### FIELD OF THE INVENTION

The present invention relates generally to medical fluid-delivery devices, and specifically to medical fluid-delivery devices that pump fluid to a subject by pressing on a conduit.

### BACKGROUND

Pumps are often used in the medical industry for delivering fluids, e.g., drugs, or diagnostic fluids, to subjects. One type of medical pump is an infusion pump, used to infuse a fluid into a subject's circulatory system via infusion tubing. Some infusion pumps pump fluid through the infusion tubing by repeatedly pressing, i.e., squeezing, the tubing.

### SUMMARY OF THE INVENTION

Typically, parameters of a pumping mechanism, e.g., pumping cycle rate, of a fluid-delivery device, e.g., an infusion pump, are calibrated for a given delivery flow rate based on a diameter of the infusion tubing. For example, in an infusion pump where fluid is pumped to the subject by repeatedly squeezing the tubing with a pressing surface, the volume of fluid that is displaced during each intake/delivery cycle of the pumping mechanism is affected by the tube size. For infusion tubing of different sizes, parameters such as a speed at which the pressing surface squeezes the tubing, a wait-time between each pumping cycle in order to let the tube refill with fluid from a fluid source (e.g., an IV bag), upper and lower limits of each pressing surface stroke, and/or parameters of a pressure sensing mechanism within the fluid-delivery device, will vary for different desired delivery flow rates.

The inventors have realized that a volume of fluid displaced within the infusion tubing for each squeeze is dependent on a relationship between (a) how far the tube is squeezed from a first position to a second position (i.e., the difference in height of the tube before and after the tube is squeezed), and (b) the unsqueezed, fully-round, outer diameter of the tubing. In order to deliver fluid at a given flow rate, the pumping cycle rate of the pumping mechanism is calibrated based on the volume of fluid displaced within the infusion tubing for each squeeze, i.e., for each pumping cycle. Thus, in order to calibrate the pumping mechanism for a given flow rate, the fully round outer diameter of the tube should be known.

Therefore, in accordance with some applications of the present invention, apparatus and methods are presented for measuring the outer diameter of a conduit, e.g., infusion tubing, within a fluid-delivery device, e.g., an infusion pump. The fluid-delivery device measuring an outer diameter of any conduit received within it allows for a "tube-agnostic" system that is not limited to only receiving a conduit of a specific diameter, but, instead, may self-calibrate parameters such as pressing surface speed, pumping cycle rate, and/or pressure mechanism parameters, based on measuring the outer diameter of the conduit within the fluid-delivery device.

There is therefore provided, in accordance with some applications of the present invention, a method for measuring a size of a fluid-filled conduit in a fluid-delivery device, the method including:
(A) isolating a segment of the fluid-filled conduit by occluding a first site of the fluid-filled conduit and a second site of the fluid-filled conduit, the isolated segment being between the first and second sites;
(B) iteratively increasing pressure within the isolated segment, by incrementally squeezing a portion of the isolated segment of the fluid-filled conduit;
(C) for each iteration of squeezing the portion of the isolated segment of the fluid-filled conduit, measuring an increase in force exerted by the isolated segment of the fluid-filled conduit, associated with a respective pressure change during that incremental squeezing; and
(D) measuring an indication of the size of the conduit when an increase in force exerted by the isolated segment, measured in response to an incremental squeezing of the portion of the isolated segment of the fluid-filled conduit, passes above a threshold value.

For some applications, measuring the increase in force includes, using a force sensor, measuring the increase in force exerted, on the force sensor, by the isolated segment of the fluid-filled conduit, during each incremental squeezing.

For some applications:
incrementally squeezing a portion of the isolated segment includes using a pressing surface to incrementally squeeze the isolated segment, and
measuring the increase in force includes, using a force sensor coupled to the pressing surface, measuring the increase in force exerted, on the pressing surface, by the fluid-filled conduit, during each incremental squeezing.

For some applications, measuring the indication of the size of the conduit includes measuring an indication of an outer diameter of the conduit.

For some applications, the method further includes inhibiting the start of fluid-delivery to a subject if the measured indication of size of the conduit indicates that the size of the conduit is not within a predetermined range of sizes.

For some applications, measuring the indication of the size of the fluid-filled conduit includes measuring the indication of the size of the fluid-filled conduit using a size sensor.

For some applications, measuring the indication of the size of the fluid-filled conduit using the size sensor includes measuring the indication of the size of the fluid-filled conduit using a size sensor that is maintained in contact with the isolated segment of the fluid-filled conduit.

For some applications, the size sensor is maintained in contact with the isolated segment of the fluid-filled conduit by a spring, the spring causing the size sensor to exert a force on the isolated segment of the fluid-filled conduit prior to the squeezing.

For some applications, measuring the increase in force includes using the size sensor to measure the force exerted, on the size sensor, by the isolated segment of the fluid-filled conduit, during the squeezing.

For some applications, measuring the indication of the size of the conduit using the size sensor includes measuring the indication of the size of the conduit using a size sensor that does not contact the conduit.

For some applications, measuring the indication of the size of the conduit using the size sensor that does not contact the isolated segment of the fluid-filled conduit includes measuring the indication of the size of the conduit using an optical sensor.

For some applications, the method further includes regulating a parameter of the fluid-delivery device in response to the measured indication of the size of the conduit.

For some applications, regulating the parameter of the fluid-delivery device includes regulating a pumping cycle rate of the fluid-delivery device.

For some applications, regulating the parameter of the fluid-delivery device includes regulating an upper limit and a lower limit of a stroke of the pressing surface of each pumping cycle.

For some applications, regulating the parameter of the fluid-delivery device includes regulating a wait-time between consecutive pumping cycles.

There is further provided, in accordance with some applications of the present invention, a method for measuring a size of a fluid-filled conduit in a fluid-delivery device, the method including:
(A) isolating a segment of the fluid-filled conduit by occluding a first site of the fluid-filled conduit and a second site of the fluid-filled conduit, the isolated segment being between the first and second sites;
(B) iteratively increasing pressure within the isolated segment, by incrementally squeezing a portion of the isolated segment of the fluid-filled conduit;
(C) for each iteration of squeezing the portion of the isolated segment of the fluid-filled conduit, measuring an increase in size of the isolated segment of the fluid-filled conduit, associated with a respective pressure change during that incremental squeezing; and
(D) measuring an indication of the size of the conduit when an increase in size of the isolated segment, measured in response to an incremental squeezing of the portion of the isolated segment of the fluid-filled conduit, passes below a threshold value.

For some applications, measuring the indication of the size of the conduit includes measuring an indication of an outer diameter of the conduit.

For some applications, the method further includes inhibiting the start of fluid-delivery to a subject if the measured indication of size of the conduit indicates that the size of the conduit is not within a predetermined range of sizes.

For some applications, the method further includes regulating a parameter of the fluid-delivery device in response to the measured indication of the size of the conduit.

For some applications, regulating the parameter of the fluid-delivery device includes regulating a pumping cycle rate of the fluid-delivery device.

For some applications, regulating the parameter of the fluid-delivery device includes regulating an upper limit and a lower limit of a stroke of the pressing surface of each pumping cycle.

For some applications, regulating the parameter of the fluid-delivery device includes regulating a wait-time between consecutive pumping cycles.

There is further provided, in accordance with some applications of the present invention, a method for measuring a size of a fluid-filled conduit in a fluid-delivery device, the method including:
isolating a segment of the fluid-filled conduit by occluding a first site of the fluid-filled conduit and a second site of the fluid-filled conduit, the isolated segment being between the first and second sites;
squeezing a portion of the isolated segment of the fluid-filled conduit;
measuring a force exerted by the isolated segment of the fluid-filled conduit during the squeezing; and
when the measured force passes above a threshold value, measuring an indication of a size of the conduit.

For some applications, measuring the indication of the size of the conduit includes measuring an indication of an outer diameter of the conduit.

For some applications, the method further includes inhibiting the start of fluid-delivery to a subject if the measured indication of size of the conduit indicates that the size of the conduit is not within a predetermined range of sizes.

For some applications, measuring the force includes, using a force sensor, measuring the force exerted, on the force sensor, by the isolated segment of the fluid-filled conduit, during the squeezing.

For some applications:
squeezing a portion of the isolated segment includes using a pressing surface to squeeze the isolated segment, and
measuring the force includes, using a force sensor coupled to the pressing surface, measuring the force exerted, on the pressing surface, by the fluid-filled conduit, during the squeezing.

For some applications, the method further includes regulating a parameter of the fluid-delivery device in response to the measured indication of the size of the fluid-filled conduit.

For some applications, regulating the parameter of the fluid-delivery device includes regulating a pumping cycle rate of the fluid-delivery device.

For some applications, regulating the parameter of the fluid-delivery device includes regulating a pumping cycle rate of the fluid-delivery device.

For some applications, regulating the parameter of the fluid-delivery device includes regulating an upper limit and a lower limit of a stroke of the pressing surface of each pumping cycle.

For some applications, regulating the parameter of the fluid-delivery device includes regulating a wait-time between consecutive pumping cycles.

For some applications, measuring the indication of the size of the fluid-filled conduit includes measuring the indication of the size of the fluid-filled conduit using a size sensor.

For some applications, measuring the indication of the size of the fluid-filled conduit using the size sensor includes measuring the indication of the size of the fluid-filled conduit using a size sensor that is maintained in contact with the isolated segment of the fluid-filled conduit.

For some applications, the size sensor is maintained in contact with the isolated segment of the fluid-filled conduit by a spring, the spring causing the size sensor to exert a force on the isolated segment of the fluid-filled conduit prior to the squeezing.

For some applications, measuring the force includes using the size sensor to measure the force exerted, on the size sensor, by the isolated segment of the fluid-filled conduit, during the squeezing.

For some applications, measuring the indication of the size of the conduit using the size sensor includes measuring the indication of the size of the conduit using a size sensor that does not contact the conduit.

For some applications, measuring the indication of the size of the conduit using the size sensor that does not contact the isolated segment of the fluid-filled conduit includes measuring the indication of the size of the conduit using an optical sensor.

There is further provided, in accordance with some applications of the present invention, apparatus for delivering a fluid to a subject, the apparatus including:
a fluid-delivery device configured to receive a conduit, the fluid-delivery device including:
   a pressing surface configured to squeeze the conduit;
   an upstream valve located upstream of the pressing surface and configured to reversibly occlude the conduit upstream of the pressing surface;
   a downstream valve located downstream of the pressing surface and configured to reversibly occlude the conduit downstream of the pressing surface;
   a force sensor positioned so as to measure an increase in force exerted by the conduit on the force sensor when the pressing surface is driven to squeeze an isolated segment of the conduit while the upstream and downstream valves are occluding the conduit on respective sides of the isolated segment; and
   a size sensor configured to measure an indication of a size of the conduit when the measured increase in force passes above a threshold value.

For some applications, the size sensor is configured to measure an indication of an outer diameter of the conduit.

For some applications, the force sensor is positioned such that, when the conduit is received within the fluid-delivery device, the force sensor is preloaded against the isolated segment of the conduit.

For some applications, the fluid-delivery device is configured to receive a conduit having an outer diameter selected from a predetermined range of outer diameters.

For some applications, the predetermined range of outer diameters includes, at least, 3 - 6 mm.

For some applications, the size sensor is positioned such that, when the conduit is received within the fluid-delivery device, the size sensor is in contact with the isolated segment of the conduit.

For some applications, the apparatus further includes a spring coupled to the size sensor such that the size sensor is maintained in contact with the isolated segment of the fluid-filled conduit by a compression force that (a) compresses the spring and (b) is caused by the conduit being received within the fluid-delivery device.

For some applications, the size sensor is configured to measure the indication of the size of the conduit when the measured increase in force passes above a threshold value that is greater than the compression force.

For some applications, the size sensor is configured to measure the indication of the size of the conduit without contacting the conduit.

For some applications, the size sensor is an optical sensor.

There is further provided, in accordance with some applications of the present invention, apparatus for delivering a fluid to a subject, the apparatus including:
a fluid-delivery device configured to receive a conduit, the fluid-delivery device including:
a pressing surface configured to squeeze the conduit;
an upstream valve located upstream of the pressing surface and configured to reversibly occlude the conduit upstream of the pressing surface;
a downstream valve located downstream of the pressing surface and configured to reversibly occlude the conduit downstream of the pressing surface; and
a sensor positioned so as to
   (a) measure an increase in force exerted by the conduit on the sensor when the pressing surface is driven to squeeze an isolated segment of the conduit while the upstream and downstream valves are occluding the conduit on respective sides of the isolated segment, and
   (b) measure an indication of a size of the conduit when the measured increase in force passes above a threshold value.

For some applications, the sensor is configured to measure an indication of an outer diameter of the conduit.

For some applications, the fluid-delivery device is configured to receive a conduit having an outer diameter selected from a predetermined range of outer diameters.

For some applications, the predetermined range of outer diameters includes, at least, 3 - 6 mm.

For some applications, the sensor is positioned such that, when the conduit is received within the fluid-delivery device, the sensor is maintained in contact with the isolated segment of the fluid-filled conduit.

For some applications, the apparatus further includes a spring coupled to the sensor such that the sensor is maintained in contact with the isolated segment of the fluid-filled conduit by a compression force that (a) compresses the spring and (b) is caused by the fluid-filled conduit being received within the fluid-delivery device.

The present invention will be more fully understood from the following detailed description of applications thereof, taken together with the drawings, in which:

### BRIEF DESCRIPTION OF THE DRAWINGS

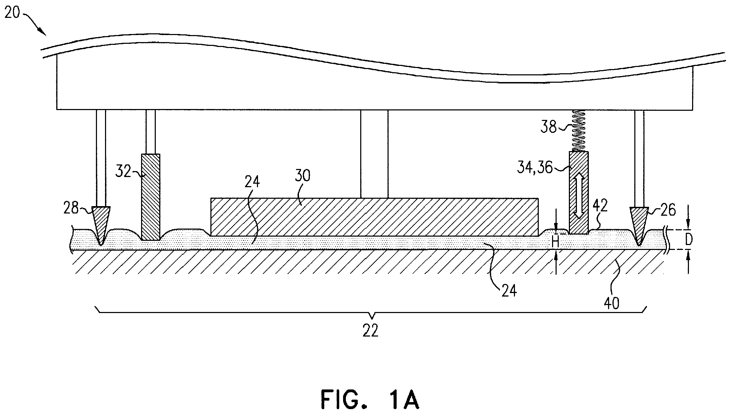

Figs. 1A-B are schematic illustrations of a fluid-delivery device, a segment of fluid-filled conduit disposed within the fluid-delivery device and isolated by an upstream valve and a downstream valve, a pressing surface for squeezing the conduit, a force sensor preloaded against the conduit, and a size sensor in contact with the conduit, in accordance with some applications of the present invention;

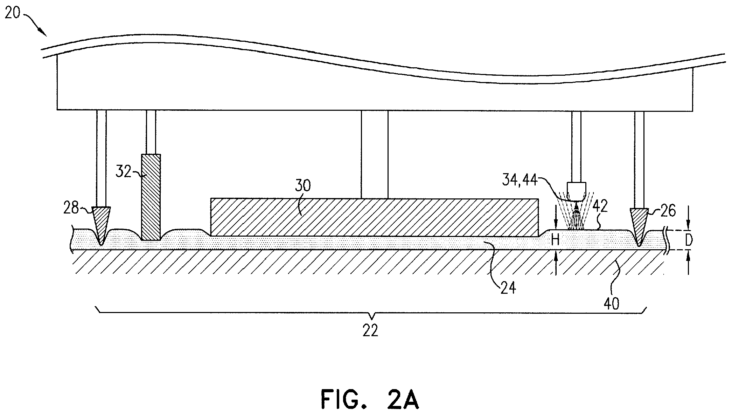

Figs. 2A-B are schematic illustrations of a fluid-delivery device, a segment of fluid-filled conduit disposed within the fluid-delivery device, and isolated by an upstream valve and a downstream valve, a pressing surface for squeezing the conduit, a force sensor, and a size sensor not in contact with the conduit, in accordance with some applications of the present invention;

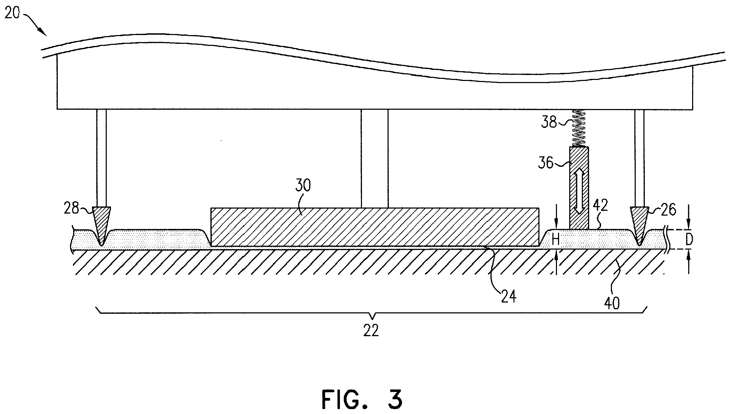

Fig. 3 is a schematic illustration of a fluid-delivery device, a segment of fluid-filled conduit disposed within the fluid-delivery device isolated by an upstream valve and a downstream valve, a pressing surface for squeezing the conduit, and a sensor configured to measure both force and displacement, in accordance with some applications of the present invention;

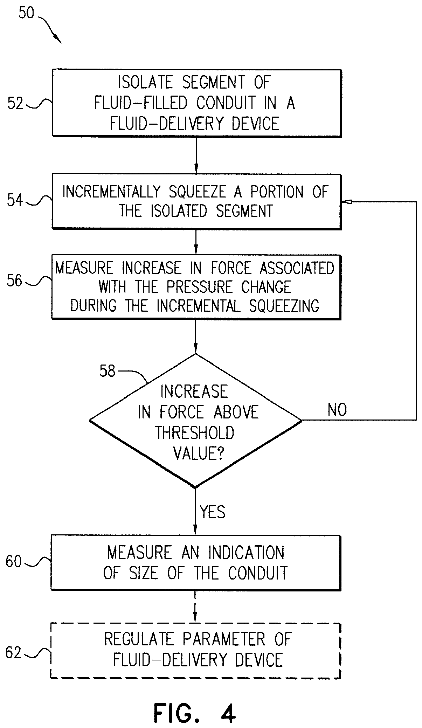

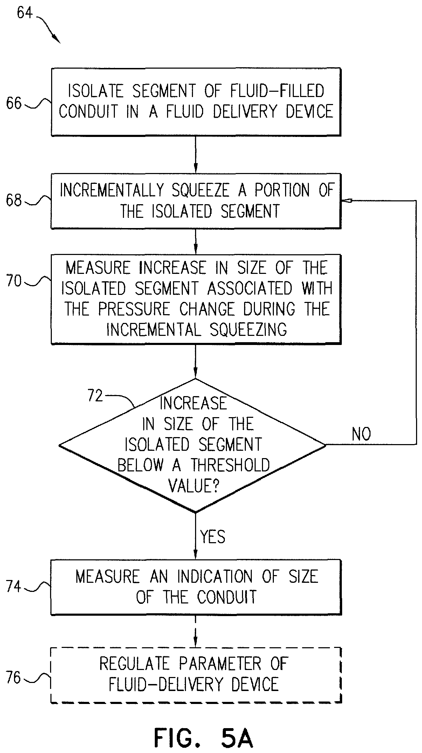

Figs. 4 and 5A-B are flowcharts showing different respective methods for measuring a size of a fluid-filled conduit in a fluid-delivery device, in accordance with some applications of the present invention; and

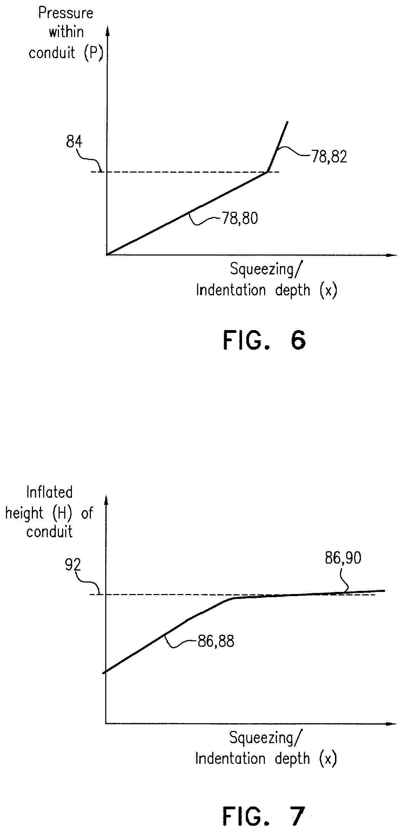

Figs. 6-7 are graphs corresponding, respectively, to the flowcharts of Figs. 4 and 5A-B.

### DETAILED DESCRIPTION

Reference is now made to Figs. 1A-B, which are schematic illustrations of a fluid-delivery device 20, a segment 22 of fluid-filled conduit 24 disposed within fluid-delivery device 20 and isolated by an upstream valve 26 and a downstream valve 28, a pressing surface 30 for squeezing conduit 24, a force sensor 32, and a size sensor 34 in contact with conduit 24, in accordance with some applications of the present invention. For some applications, fluid-delivery device 20 receives a conduit 24 having a fully-round diameter D, e.g., outer diameter D of conduit 24, selected from a predetermined range of outer diameters. For example, standard outer diameters of infusion tubing may range from 3 - 6 mm, with the internal diameter typically being 0.8 mm to 2 mm smaller than the outer diameter. Upstream valve 26 and downstream valve 28 may be activated to reversibly occlude conduit 24. An isolated segment 22 of conduit 24 is defined when both upstream valve 26 and downstream valve 28 are occluding conduit 24 at the same time. Typically, prior to isolating segment 22 of conduit 24, conduit 24 is filled with fluid, e.g., liquid, such that the contents of conduit 24 are generally incompressible. Thus, pressing surface 30 may be used to squeeze a portion of isolated segment 22, which in turn causes other portions of isolated segment 22 that are not being squeezed by pressing surface 30 to inflate.

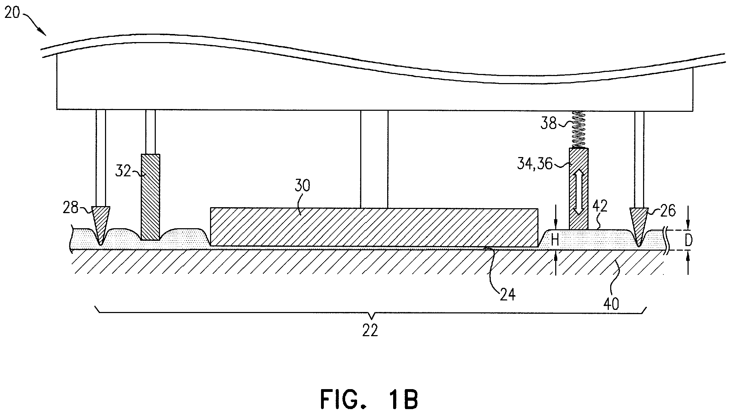

Force sensor 32 is positioned so as to measure an increase in force exerted by conduit 24 on force sensor 32 as pressing surface 30 is driven to squeeze isolated segment 22 of conduit 24, such as is shown in Fig. 1B. For some applications, pressing surface 30 is driven to iteratively squeeze isolated segment 22, thereby incrementally increasing pressure within isolated segment 22. For each incremental increase in pressure, due to each incremental squeeze, force sensor 32 measures an increase in force exerted by conduit 24 on force sensor 32. When the increase in force exerted by conduit 24 on force sensor 32 for each incremental squeeze passes above a threshold value, it can be assumed that an outer diameter of the now-inflated portions of isolated segment 22 represents fully-round outer diameter D of conduit 24. Once the now-inflated portions of isolated segment 22 are fully inflated, i.e., have reached fully-round outer diameter D, the wall of conduit 24 starts to provide stronger resistance against further inflation of isolated segment 22. This causes the change in pressure within isolated segment 22 per each incremental squeeze to suddenly increase, defining the threshold value (further described hereinbelow with reference to Fig. 6). Thus, when the increase in force passes above the threshold value, size sensor 34 measures an indication of size, e.g., an indication of outer diameter, e.g., the diameter, of conduit 24. Typically, force sensor 32 is positioned so as to be preloaded against isolated segment 22 of conduit 24 so as to increase sensitivity of the force measurement, such as is shown in Fig. 1A.

For some applications, force sensor 32 measures an absolute value of force exerted by conduit 24 on force sensor 32, and when the value of the measured force is high enough, i.e., once the force reaches the predetermined threshold value, it can be assumed that an outer diameter of the now-inflated portions of isolated segment 22 represents fully-round outer diameter D of conduit 24. Thus, when the measured force exerted on force sensor 32 reaches the threshold value, size sensor 34 measures an indication of size, e.g., an indication of outer diameter, e.g., the outer diameter, of conduit 24. Typically, force sensor 32 is positioned so as to be preloaded against isolated segment 22 of conduit 24 so as to increase sensitivity of the force measurement, such as is shown in Fig. 1A.

For some applications, size sensor 34 may be a contact sensor 36, positioned so as to be in contact with isolated segment 22 of conduit 24 when conduit 24 is received within fluid-delivery device 20. For some applications, contact sensor 36 may comprise a spring 38 that is coupled to contact sensor 36 so as to maintain contact sensor 36 in contact with isolated segment 22 of conduit 24, regardless of the size of the conduit received within fluid-delivery device 20. For example, spring 38 may bias contact sensor 36 towards a lower surface 40, against which conduit 24 is braced when received within fluid-delivery device 20. As shown in Fig. 1A, due to spring 38 biasing contact sensor 36 towards lower surface 40, contact sensor 36 itself slightly squeezes conduit 24. Thus, when conduit 24 is received within fluid-delivery device 20, conduit 24 causes a compression force that compresses spring 38 a first compression distance. Now-compressed spring 38 ensures maintained contact between contact sensor 36 and isolated segment 22 with a compression force. Typically, a larger-sized conduit 24 will cause a larger compression force than a compression force caused by a smaller-sized conduit 24.

For some applications, contact sensor 36 measures the indication of the size of conduit 24, e.g., the indication of fully-round outer diameter D of conduit 24, by measuring a height H of the top of outer wall 42 of isolated segment 22 with respect to lower surface 40. Alternatively, contact sensor 36 may be calibrated so as to measure the indication of fully-round outer diameter D by measuring a height of some other reference point on conduit 24 with respect to lower surface 40. For some applications, when conduit 24 is received within fluid-delivery device 20, contact sensor 36 may measure a height H of the top of outer wall 42 of isolated segment 22, based on the first compression distance of spring 38, prior to isolated segment 22 being squeezed by pressing surface 30. As shown in Fig. 1A, prior to isolated segment 22 being squeezed by pressing surface 30, height H of the top of outer wall 42 of isolated segment 22 is therefore typically less than fully-round outer diameter D. Subsequently, during the squeezing of isolated segment 22, the inflation of isolated segment 22 causes a vertical displacement of contact sensor 36, which in turn causes further compression of spring 38. The further compression distance of spring 38 corresponds to a displacement of contact sensor 36 during the squeezing, the displacement of contact sensor 36 being indicative of the now-inflated height H of the top of outer wall 42 of isolated segment 22. Spring 38 is typically compliant enough so as to allow the inflation of isolated segment 22 to compress spring 38 with relative ease.

As described hereinabove, during the squeezing of isolated segment 22 it can be assumed that (a) an outer diameter of the now-inflated portions of isolated segment 22 represents a fully-round outer diameter D of conduit 24 when (b) the force being measured by force sensor 32 reaches a threshold value. Thus, when the measured force reaches the threshold value, it may be assumed that the measured displacement of contact sensor 36 during the squeezing, which is indicative of the now-inflated height H of the top of outer wall 42 of isolated segment 22, is indicative of the fully-round outer diameter D of conduit 24.

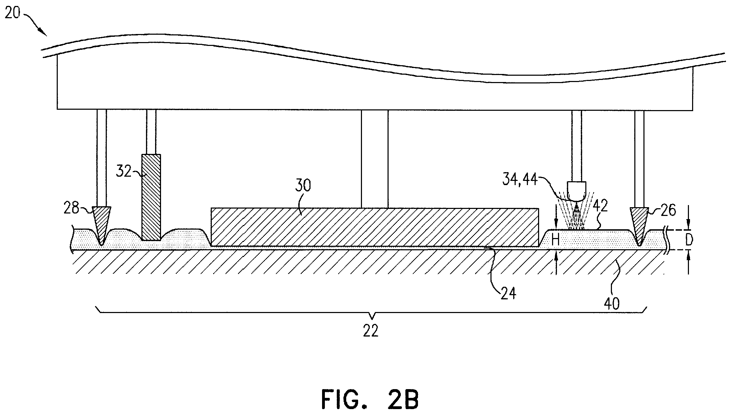

Reference is now made to Figs. 2A-B, which are schematic illustrations of fluid-delivery device 20, segment 22 of fluid-filled conduit 24 disposed within fluid-delivery device 20 and isolated by upstream valve 26 and downstream valve 28, pressing surface 30 for squeezing conduit 24, force sensor 32, and size sensor 34 not in contact with conduit 24, in accordance with some applications of the present invention. For some applications, size sensor 34 may be a non-contact size sensor that does not contact isolated segment 22 of conduit 24. Fig. 2A shows pressing surface 30 in starting position, and Fig. 2B shows pressing surface 30 squeezing conduit 24. In the absence of size sensor 34 pressing against conduit 24 (such as is shown in Figs. 1A-B), the difference in height H of the top of outer wall 42 of isolated segment 22 before and after conduit 24 is squeezed is typically small, e.g., on the order of magnitude of microns, and is therefore not portrayed in the progression from Fig. 2A to Fig. 2B. For some applications, the difference in height H of the top of outer wall 43 of isolated segment 22 before and after conduit 24 is squeezed may be larger due to parameters such as elasticity of the conduit, distance between the upstream and downstream valves, length of pressing surface 30, and depth of the pressing surface stroke.

For example, size sensor 34 may be an optical sensor 44. Optical sensor 44 typically measures height H of the top of outer wall 42 of isolated segment 22 relative to a refence point that is determined during an initial calibration of fluid-delivery device 20, e.g., during production of fluid-delivery device 20. The reference point may be lower surface 40 against which conduit 24 is braced when conduit 24 is received within fluid-delivery device 20. Alternatively or additionally, the reference point may be a different surface within fluid-delivery device 20, whose distance from optical sensor 44 is determined during initial calibration. As described hereinabove, once the force exerted by conduit 24 on force sensor 32 reaches the predetermined threshold value, optical sensor 44 may measure height H of the top of outer wall 42 of isolated segment 22, and it can be assumed that the now-inflated height H represents fully-round outer diameter D of conduit 24.

Reference is now made to Fig. 3, which is a schematic illustration of fluid-delivery device 20, segment 22 of fluid-filled conduit 24 disposed within fluid-delivery device 20 and isolated by upstream valve 26 and downstream valve 28, pressing surface 30 for squeezing conduit 24, and a sensor configured to measure both force and displacement, in accordance with some applications of the present invention. For some applications, instead of a separate force sensor and a separate size sensor, contact sensor 36 may be used to measure (a) the force exerted by conduit 24 on contact sensor 36 during the squeezing, and (b) the indication of the size of conduit 24, e.g., of fully-round outer diameter D of conduit 24, when the measured force reaches the predetermined threshold value.

As described hereinabove, spring 38 of contact sensor 36 may be coupled to contact sensor 36 so as to maintain contact sensor 36 in contact with conduit 24 when conduit 24 is received within fluid-delivery device 20. As pressing surface 30 squeezes isolated segment 22 of conduit 24, contact sensor 36 is vertically displaced causing compression of spring 38. This compression is converted to a measurement of the force exerted by conduit 24 on contact sensor 36. When the measured force reaches the predetermined threshold, as described hereinabove, it may be assumed that the displacement of contact sensor 36 during the squeezing is indicative of fully-round outer diameter D of conduit 24, and as such, the same compression of spring 38 that resulted in the measured force reaching the threshold value is converted to a displacement measurement. Thus, the inflated height H of the top of outer wall 42 of isolated segment 22 is measured, the inflated height H being indicative of fully-round outer diameter D of conduit 24.

For some applications, force sensor 32 may also be coupled to, e.g., mounted on, pressing surface 30, such that as pressing surface 30 squeezes isolated segment 22 of conduit 24, the force sensor measures the force exerted by conduit 24 on pressing surface 30. For example, force sensor may be mounted on the side of pressing surface 30, or between pressing surface 30 and conduit 24. As described hereinabove, when the measured force reaches the predetermined threshold value, size sensor 34 measures the indication of the size, e.g., fully-round outer diameter D, of conduit 24.

It is noted that in Figs. 1B, 2B, and 3, isolated segment 22 of conduit 24 is shown as having reached its fully-round outer diameter D due to the squeezing, at which point height H of the top of outer wall 42 of isolated segment 22 is equal to fully round outer diameter D of conduit 24. Thus, height H of the top of outer wall 42 of isolated segment 22 appears in the figures to be the same as fully-round outer diameter D.

Additionally, it is noted that while the description above, with reference to Figs. 1A-B and Fig. 3, relates to compression of spring 38, the scope of the present invention includes spring 38 being positioned within fluid-delivery device 20 such that spring 38 is attached to the conduit 24, e.g., looped around conduit 24, in a way that would cause spring 38 to stretch (rather than compress) upon inflation of conduit 24.

Reference is now made to Fig. 4, which is a flowchart depicting a method 50 for measuring the size, e.g., fully-round outer diameter D, of fluid-filled conduit 24, in accordance with some applications of the present invention. For some applications, as described hereinabove with reference to Figs. 1-3, method 50 is based on a measurement of an increase in force exerted by conduit 24 on an element external to conduit 24, as conduit 24 is squeezed. In a first step 52 of method 50, segment 22 of conduit 24 is isolated by occluding a first site of conduit 24 and a second site of conduit 24, for example using upstream valve 26 and downstream valve 28 respectively, such that isolated segment 22 is between the first and second sites. Pressure within isolated segment 22 is then incrementally increased by incrementally squeezing a portion of isolated segment 22 (step 54) so as to inflate portions of isolated segment 22 that are not being squeezed.

For each incremental squeezing of isolated segment 22, an increase in force exerted by isolated segment 22 of conduit 24 associated with the increase in pressure during the incremental squeezing is measured (step 56). For some applications, the increase in force may be measured with a dedicated force sensor, such as force sensor 32 as described with reference to Figs. 1A-B and Figs. 2A-B. Alternatively, for some applications, there may not be a sensor dedicated to only measuring force, and the force may be measured using the same sensor that is used to measure the size of conduit 24, such as size sensor 34, e.g., contact sensor 36, as described with reference to Fig. 3. Optionally, a force sensor may be coupled to pressing surface 30, and the force exerted by conduit 24 on pressing surface 30 during the squeezing is measured directly by pressing surface 30 as it squeezes conduit 24.

As described hereinabove, when the measured increase in force passes above a predetermined threshold value (as depicted by decision diamond 58 in Fig. 4, and as further described hereinbelow with reference to Fig. 6), an indication of the size, e.g., fully-round outer diameter D, of conduit 24 is measured (step 60). The indication of the size, e.g., fully-round outer diameter D, of conduit 24 may be measured by a size sensor that contacts conduit 24, e.g., contact sensor 36 as described with reference to Figs. 1A-B and 3, or alternatively by a size sensor that is not in contact with conduit 24, e.g., optical sensor 44 as described with reference to Figs. 2A-B.

For some applications, a parameter of fluid-delivery device 20 may be regulated (step 62) in order to obtain a desired flow rate in response to the measured indication of the size of conduit 24. For some applications, the upper and lower limits of the pressing surface stroke are fixed, and a pumping cycle rate of the fluid-delivery device may be regulated in order to obtain a desired flow rate in response to the measured indication of size conduit 24. Alternatively, the pressing surface stroke may be adjustable, e.g., pressing surface 30 may be controlled by a lead screw and gear, and the upper and/or lower limits of each pressing surface stroke may be regulated in order to obtain a desired flow rate in response to the measured indication of size of conduit 24. For some applications, a wait-time between each pumping cycle, e.g., in order to let the tube refill with fluid from a fluid source (e.g., an IV bag), may be regulated in response to the measured indication of size of conduit 24. For some applications, parameters of a pressure sensing mechanism within the fluid-delivery device may vary based on desired flow rate, and thus may be regulated in response to the measured indication of size, e.g., fully-round outer diameter D, of conduit 24.

Reference is now made to Fig. 5A, which is a flowchart depicting a method 64 for measuring the size, e.g., fully-round outer diameter D, of fluid-filled conduit 24, in accordance with some applications of the present invention. Alternatively to method 50, which is based on force measurement, method 64 is based on watching an increase in the value indicative of the size, e.g., fully-round outer diameter D, of conduit 24, during the squeezing. In a first step 66 of method 64, segment 22 of conduit 24 is isolated by occluding a first site of conduit 24 and a second site of conduit 24, for example using upstream valve 26 and downstream valve 28 respectively, such that isolated segment 22 is between the first and second sites. Pressure within isolated segment 22 is then incrementally increased by incrementally squeezing a portion of isolated segment 22 (step 68), and an increase in size of conduit 24 associated with the increase in pressure during the incremental squeezing is measured (step 70).

As the portion of isolated segment 22 is squeezed, the portions of isolated segment 22 not being squeezed begin to inflate, as described hereinabove. At first the inflation is rapid, however as the inflated height H of isolated segment 22 nears fully-round outer diameter D, the increase in size of isolated segment 22 measured in response to an incremental squeezing of isolated segment 22 starts to slow down due to the increase in resistance from the wall of conduit 24, i.e., as the inflated height H of isolated segment 22 nears fully-round outer diameter D, the increase in size of isolated segment 22 for each incremental squeeze is reduced (as further described hereinbelow with reference to Fig. 7) . Thus, steps 68 and 70 may be iteratively repeated until the measured increase in size of conduit 24, measured in response to an incremental squeezing of the portion of isolated segment 22, passes below a threshold value. At that point it can be assumed that the now-inflated height H of the top of outer wall 42 of isolated segment 22 represents fully-round outer diameter D of conduit 24, and thus an indication of the size, e.g., fully-round outer diameter D, of conduit 24 is measured (step 74).

For some applications, the indication of the size, e.g., fully-round outer diameter D, of conduit 24 may be measured by a size sensor that contacts conduit 24, e.g., contact sensor 36 as shown in Figs. 1A-B, and 3. Alternatively, for some applications, the indication of the size, e.g., fully-round outer diameter D, of conduit 24 may be measured by a sensor that is not in contact with conduit 24, e.g., optical sensor 44 as shown in Figs. 2A-B.

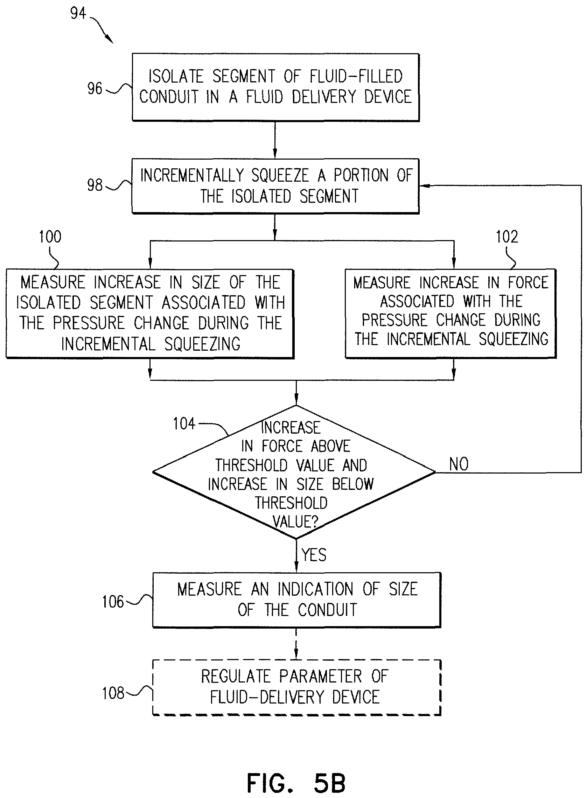

Reference is now made to Fig. 5B, which is a flowchart depicting a method 94 for measuring the size, e.g., fully-found outer diameter D, of fluid-filled conduit 24, in accordance with some applications of the present invention. Method 94 is a combination of method 50 and method 64, and is based on watching (a) the force measurement and (b) an increase in the value indicative of the size, e.g., fully-round outer diameter D, of conduit 24, during squeezing. In a first step 96 of method 94, segment 22 of conduit 24 is isolated by occluding a first site of conduit 24 and a second site of conduit 24, for example using upstream valve 26 and downstream valve 28 respectively, such that isolated segment 22 is between the first and second sites. Pressure within isolated segment 22 is then incrementally increased by incrementally squeezing a portion of isolated segment 22 (step 98). During the incremental squeezing (a) an increase in size of conduit 24 associated with the increase in pressure is measured (step 100) and (b) an increase in force exerted by isolated segment 22 of conduit 24 associated with the increase in pressure during the incremental squeezing is measured (step 102). The increase in force may be measured using the same techniques as described hereinabove with reference to Fig. 4.

In contrast to method 50 and method 64, each of which relies on one threshold being met in order to determine when the size measurement of conduit 24 should be taken, method 94 relies on both (a) the increase in size threshold and (b) the increase in force threshold, being met. Thus, when (a) the measured increase in size of conduit 24, measured in response to the incremental squeezing of the portion of isolated segment 22, passes below a threshold value, and (b) the measured increase in force, measured in response to the incremental squeezing of the portion of isolated segment 22, passes above a predetermined threshold value (as depicted by decision diamond 104 in Fig. 5B), an indication of the size, e.g., fully-round outer diameter D, of conduit 24 is measured (step 106).

For some applications, a parameter of fluid-delivery device 20 may be regulated (step 108) in response to the measured indication of the size of conduit 24, as described hereinabove.

Reference is now made to Fig. 6, which is a graph corresponding to the method depicted in Fig. 4, in accordance with some applications of the present invention. Curve 78 on the graph represents a model of the pressure P within isolated segment 22 as pressing surface 30 incrementally squeezes, i.e., indents by a distance x, isolated segment 22. The slope of curve 78 represents the rate of change of pressure P within isolated segment 22 as pressing surface 30 incrementally squeezes isolated segment 22. Thus, the slope of curve 78 can be described as dP/dx.

The slope of segment 80 of curve 78 represents the rate of change in internal pressure before the now-inflated portions of isolated segment 22 reach fully-round outer diameter D, and the slope of segment 82 of curve 78 represents the rate of change in internal pressure once the now-inflated portions of isolated segment 22 reach fully-round outer diameter D. As described hereinabove, once the now-inflated portions of isolated segment 22 are fully inflated, the wall of conduit 24 starts providing increased resistance against further inflation, typically causing a relatively sharp increase in the rate of change of pressure within conduit 24 per each further incremental squeeze. Thus, as depicted in the graph of Fig. 6, the slope of segment 80 represents a first rate of change of internal pressure (dP1/dx), and the slope of segment 82 represents a second rate of change of internal pressure (dP2/dx). The threshold value for assuming that the now-inflated portions of isolated segment 22 have now reached fully-round outer diameter D is typically when dP/dx increases from dP1/dx to dP2/dx, represented by dashed line 84.

Reference is now made to Fig. 7, which is a graph corresponding to the method depicted in Fig. 5, in accordance with some applications of the present invention. Curve 86 on the graph represents a model of the inflated height H of isolated segment 22 as pressing surface 30 incrementally squeezes, i.e., indents by a distance x, isolated segment 22. The slope of curve 86 represents the rate of change of inflated height H of isolated segment 22 as pressing surface 30 incrementally squeezes isolated segment 22. Thus, the slope of curve 86 can be described as dH/dx.

The slope of segment 88 of curve 86 represents the rate of change in inflated height H before inflated height H of isolated segment 22 nears fully-round outer diameter D, and the slope of segment 90 of curve 86 represents the rate of change in inflated height H once inflated height H of isolated segment 22 reaches fully-round outer diameter D. As described hereinabove, as the inflated height H of isolated segment 22 nears fully-round outer diameter D, the increase in size of isolated segment 22 measured in response to an incremental squeezing of isolated segment 22 starts to slow down and becomes somewhat asymptotic. Thus, as depicted in the graph of Fig. 7, the slope of segment 88 represents a first rate of change of inflated height H (dH1/dx), and the slope of segment 90 represents a second rate of change of inflated height H (dH2/dx). The threshold value for assuming that the now-inflated portions of isolated segment 22 have now reached fully-round outer diameter D is typically when dH/dx decreases from dH1/dx to dH2/dx, represented by dashed line 92. For some applications, the threshold value for assuming that the now-inflated portions of isolated segment 22 have now reached fully-round outer diameter D may be when the rate of change in inflated height H reaches approximately zero, i.e., dH/dx ∼ 0.

For some applications, a parameter of fluid-delivery device 20 may be regulated (step 76) in response to the measured indication of the size of conduit 24, as described hereinabove.

For some applications, fluid-delivery device 20 may inhibit delivery of fluid to a subject if the measured indication of size of conduit 24 indicates that conduit 24 is not within a predetermined range of sizes. For example, if an infusion tube placed into fluid-delivery device 20 is measured to be either too small (e.g., less than 3 mm in outer diameter) or too large (e.g., greater than 6 mm in outer diameter), e.g., not within 3 - 6 mm in outer diameter, then fluid-delivery device 20 will not start a treatment of fluid-delivery to the subject.

It will be appreciated by persons skilled in the art that the present invention is not limited to what has been particularly shown and described hereinabove. Rather, the scope of the present invention includes both combinations and subcombinations of the various features described hereinabove, as well as variations and modifications thereof that are not in the prior art, which would occur to persons skilled in the art upon reading the foregoing description.

## Claims

1. A method for measuring a size of a fluid-filled conduit in a fluid-delivery device, the method comprising:
(A) isolating a segment of the fluid-filled conduit by occluding a first site of the fluid-filled conduit and a second site of the fluid-filled conduit, the isolated segment being between the first and second sites;
(B) iteratively increasing pressure within the isolated segment, by incrementally squeezing a portion of the isolated segment of the fluid-filled conduit; and
either:
(C) for each iteration of squeezing the portion of the isolated segment of the fluid-filled conduit, measuring an increase in force exerted by the isolated segment of the fluid-filled conduit, associated with a respective pressure change during that incremental squeezing; and
(D) measuring an indication of the size of the conduit when an increase in force exerted by the isolated segment, measured in response to an incremental squeezing of the portion of the isolated segment of the fluid-filled conduit, passes above a threshold value;
or:
(E) for each iteration of squeezing the portion of the isolated segment of the fluid-filled conduit, measuring an increase in size of the isolated segment of the fluid-filled conduit, associated with a respective pressure change during that incremental squeezing; and
(F) measuring an indication of the size of the conduit when an increase in size of the isolated segment, measured in response to an incremental squeezing of the portion of the isolated segment of the fluid-filled conduit, passes below a threshold value.

2. The method according to claim 1, wherein measuring the increase in force comprises, using a force sensor, measuring the increase in force exerted, on the force sensor, by the isolated segment of the fluid-filled conduit, during each incremental squeezing.

3. The method according to claim 1, wherein:
incrementally squeezing a portion of the isolated segment comprises using a pressing surface to incrementally squeeze the isolated segment, and
measuring the increase in force comprises, using a force sensor coupled to the pressing surface, measuring the increase in force exerted, on the pressing surface, by the fluid-filled conduit, during each incremental squeezing.

4. The method according to claim 1, wherein measuring the indication of the size of the conduit comprises measuring an indication of an outer diameter of the conduit.

5. The method according to any one of claims 1-4, wherein measuring the indication of the size of the fluid-filled conduit comprises measuring the indication of the size of the fluid-filled conduit using a size sensor.

6. The method according to claim 5, wherein measuring the indication of the size of the fluid-filled conduit using the size sensor comprises measuring the indication of the size of the fluid-filled conduit using a size sensor that is maintained in contact with the isolated segment of the fluid-filled conduit, and wherein the size sensor is maintained in contact with the isolated segment of the fluid-filled conduit by a spring, the spring causing the size sensor to exert a force on the isolated segment of the fluid-filled conduit prior to the squeezing.

7. The method according to claim 5, wherein measuring the indication of the size of the conduit using the size sensor comprises measuring the indication of the size of the conduit using an optical sensor.

8. The method according to any one of claims 1-4, further comprising regulating a parameter of the fluid-delivery device in response to the measured indication of the size of the conduit.

9. The method according to claim 8, wherein regulating the parameter of the fluid-delivery device comprises regulating a pumping cycle rate of the fluid-delivery device.

10. The method according to claim 8, wherein regulating the parameter of the fluid-delivery device comprises regulating an upper limit and a lower limit of a stroke of the pressing surface of each pumping cycle.

11. The method according to claim 8, wherein regulating the parameter of the fluid-delivery device comprises regulating a wait-time between consecutive pumping cycles.

12. Apparatus for delivering a fluid to a subject, the apparatus comprising:
a fluid-delivery device configured to receive a conduit, the fluid-delivery device comprising:
a pressing surface configured to squeeze the conduit;
an upstream valve located upstream of the pressing surface and configured to reversibly occlude the conduit upstream of the pressing surface;
a downstream valve located downstream of the pressing surface and configured to reversibly occlude the conduit downstream of the pressing surface; and
(A) a force sensor positioned so as to measure an increase in force exerted by the conduit on the force sensor when the pressing surface is driven to squeeze an isolated segment of the conduit while the upstream and downstream valves are occluding the conduit on respective sides of the isolated segment; and
a size sensor configured to measure an indication of a size of the conduit when the measured increase in force passes above a threshold value, or
(B) a sensor positioned so as to
(a) measure an increase in force exerted by the conduit on the sensor when the pressing surface is driven to squeeze an isolated segment of the conduit while the upstream and downstream valves are occluding the conduit on respective sides of the isolated segment, and
(b) measure an indication of a size of the conduit when the measured increase in force passes above a threshold value.

13. The apparatus according to claim 12, wherein (a) the size sensor is configured to measure an indication of an outer diameter of the conduit or (b) the sensor is configured to measure an indication of an outer diameter of the conduit.

14. The apparatus according to any one of claims 12-13, wherein (a) the force sensor is positioned such that, when the conduit is received within the fluid-delivery device, the force sensor is preloaded against the isolated segment of the conduit, or (b) the sensor is positioned such that, when the conduit is received within the fluid-delivery device, the sensor is maintained in contact with the isolated segment of the fluid-filled conduit.

15. The apparatus according to claim 14, (i) further comprising a spring coupled to the size sensor such that the size sensor is maintained in contact with the isolated segment of the fluid-filled conduit by a compression force that (a) compresses the spring and (b) is caused by the conduit being received within the fluid-delivery device, or (ii) further comprising a spring coupled to the sensor such that the sensor is maintained in contact with the isolated segment of the fluid-filled conduit by a compression force that (a) compresses the spring and (b) is caused by the fluid-filled conduit being received within the fluid-delivery device.
